(19) Europäisches Patentamt
European Patent Office
Office européen des brevets

(11) Publication number: **0 077 662**
**B1**

(12) **EUROPEAN PATENT SPECIFICATION**

(45) Date of publication of patent specification: **02.10.85**

(21) Application number: **82305502.5**

(22) Date of filing: **15.10.82**

(51) Int. Cl.[4]: **C 07 C 79/46**, A 61 K 31/215, C 07 C 101/44, C 07 D 209/48, C 07 C 99/00

(54) **Preparation of nitroarylacetic acid esters and derivatives thereof.**

(30) Priority: **16.10.81 US 312176**
**17.09.82 US 419341**
**30.04.82 US 373633**
**17.09.82 US 419344**

(43) Date of publication of application:
**27.04.83 Bulletin 83/17**

(45) Publication of the grant of the patent:
**02.10.85 Bulletin 85/40**

(84) Designated Contracting States:
**AT BE CH DE FR GB IT LI LU NL SE**

(56) References cited:
**THE JOURNAL OF ORGANIC CHEMISTRY, vol. 45, no. 8, 11th April 1980, pp. 1534-1535, The American Chemical Society, Columbus, Ohio (US); P.BEAK et al.: "Quantitative Model of Solvent Effects on Hydroxypyridine-Pyrodone and Mercaptopyridine-Thiopyridone Equilibria: Correlation with Reaction-Field and Hydrogen-bonding Effects"**

(73) Proprietor: **ETHYL CORPORATION**
**330 South Fourth Street P.O. Box 2189**
**Richmond Virginia 23219 (US)**

(72) Inventor: **Stahly, G. Patrick**
**6449 Peggy Street**
**Baton Rouge Louisiana 70808 (US)**
Inventor: **Stahly, Barbara Clack**
**6449 Peggy Street**
**Baton Rouge Louisiana 70808 (US)**

(74) Representative: **Bizley, Richard Edward et al**
**BOULT, WADE & TENNANT 27 Furnival Street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 077 662 B1

## Description

This invention relates to processes for preparing nitroarylacetic acid esters and derivatives thereof.

There are known techniques of preparing profen-type pharmaceuticals and other materials having a relatively complex aryl group attached to the alpha-carbon of a substituted or unsubstituted acetic acid. For example, U.S. patents 3,755,427 (Adams et al.), 3,959,364 (Armitage et al.) and 4,278,516 (Zaiko et al.) disclose processes for converting various starting materials into flurbiprofen, i.e., 2-(2-fluoro-4-biphenylyl)-propionic acid, and similar compounds having anti-inflammatory, analgesic, and anti-pyretic properties; Carney et al., "A Potent Non-Steroidal Anti-Inflammatory Agent: 2-[3-Chloro-4-(3-pyrrolinyl)-phenyl]propionic Acid," *Experientia*, Vol. 29, page 938 (1972) teach, inter alia, the preparation of their anti-inflammatory agent—also known as pirprofen—via an ethyl 2-(3-chloro-4-nitrobenzene)propionate intermediate; U.S. Patent 4,239,901 (Rainer) shows that pyrazol-1-ylphenyl and pyrazolin-1-ylphenylacetic acids having anti-inflammatory properties can be synthesized from various intermediates, including polychloronitrobenzeneacetic acid esters; and it is well known that indoprofen, i.e., 2-[4-(1,3-dihydro-1-oxo-2H-isoindol-2-yl)phenyl]propionic acid, and the indobufen, i.e., 2-[4-(1,3-dihydro-1-oxo-2H-isoindol-2-yl)phenyl]butyric acid, can be prepared from the appropriate 2-(4-nitrobenzene)-alphaalkylacetic acids.

Nitroarylacetic acids and their esters, as well as the amino derivatives thereof, have been found to be particularly useful intermediates for the synthesis of these pharmaceuticals. However, in the past, a disadvantage of employing them as pharmaceutical intermediates has been the difficulty of preparing them by conventional techniques. Even the preferred procedures for preparing them have proven to be difficult, tedious, and time-consuming, as evidenced by Example 23 of U.S. Patent 3,868,391 (Carney et al. II) and Example 16 of Rainer, both of which show the use of days of refluxing to accomplish only a portion of their syntheses.

It would obviously be a welcome contribution to the art to provide a method of synthesizing nitrobenzeneacetic acid esters and analogs and derivatives thereof in a simple and straightforward manner.

This invention aims to provide processes for preparing nitroarylacetic acid esters in moderate-to-good yield with high selectivity in a simple and straightforward manner: In addition, novel processes for preparing derivatives of the esters are provided.

The invention provides a process comprising reacting a nitroaromatic compound with an alpha,alpha-disubstituted acetic acid ester, in a substantially anhydrous aprotic solvent and in the presence of a base thereby forming a nitroarylacetic acid ester, and, when appropriate, converting the nitroarylacetic acid ester to a desired derivative thereof.

Nitroaromatic compounds utilizable in the practice of the invention include a variety of such compounds—the chief requirements for their utility being that (1) they bear at least one nitro substituent on an aromatic ring, (2) they contain at least one replaceable hydrogen on an aromatic ring to which a nitro group is attached, and (3) they are devoid of substituents which would interfere with the desired nucleophilic substitution reaction, e.g., substituents bearing an acidic proton, such as $—NH_2$, —OH, —SH, —NHR, etc.

Thus, the utilizable nitroaromatic compounds include compounds having one or more simple or fused aromatic rings containing five or six members and either bearing no substituents other than nitro substituents, or also bearing any of a variety of inert substituents, i.e., substituents that do not interfere with the desired nucleophilic substitution reaction, such as halo, alkyl, alkoxy, alkylmercapto, trifluoromethyl, dialkylamino, dialkanoylamino, cyano, dialkylcarbamoyl, alkylsulfonyl, dialkylsulfamoyl, alkoxyalkyl, haloalkyl, cycloalkyl, halocycloalkyl, etc.—any alkyl chains in the substituents generally being lower alkyl chains. ("Lower alkyl" is used in this specification in its usual sense to refer to an alkyl group containing up to about 6 carbons.) When the nitroaromatic compound contains more than one ring, any such inert substituent may be on the same ring as the ring bearing a nitro substituent and/or on a ring which is directly or indirectly attached to the ring bearing a nitro substituent.

When the aromatic ring bearing the required nitro substituent is a six-membered ring, there will be at least one replaceable hydrogen in a position para or ortho to the carbon bearing the nitro substituent; and it is preferred that there be a replaceable hydrogen in the para position. Nitroaromatic compounds having a five-membered ring should have a replaceable hydrogen on a carbon adjacent to, or separated by two ring atoms from, the carbon bearing the nitro substituent.

In accordance with one embodiment of the invention, the nitroaromatic compound is a compound which is devoid of halogen on the aromatic ring bearing the required nitro group. Illustrative of such compounds are heterocyclic compounds which preferably contain five or six-membered rings having aromatic character, such as nitropyridine-N-oxide, 5-nitroisoquinoline, 5- and 6-nitroquinolines, 2-nitrothiophene, etc.; fused-ring aromatic compounds, such as the 1- and 2-nitronaphthalenes, etc.; aromatic compounds containing a plurality of simple rings, such as the 2-, 3-, and 4-nitrobiphenyls, the 2-, 3-, and 4-benzylnitrobenzenes, 2-nitrodiphenyl ether, etc.; and aromatic compounds containing a single simple ring, such as nitrobenzene, 2-methylnitrobenzene, the 2,3-, 2,5-, and 3,5-dimethylnitrobenzenes, the 2,4- and 2,6-diethylnitrobenzenes, 3,4-dibutylnitrobenzene, the 1,2- and 1,3-dinitrobenzenes, 2,6-dinitro-

toluene, the 1,2,3- and 1,2,4-trinitrobenzenes, 2-nitro-N,N-diethylaniline, 4-nitro-N-ethylacetanilide, 2-nitrobenzylcyanide, 2-nitrophenyl acetate, etc.

In accordance with another embodiment of the invention, the nitroaromatic compound is a compound which bears at least one halo substituent on the aromatic ring bearing the required nitro group. Exemplary of such compounds are the 2-, 3-, and 4- chloronitrobenzenes; the 2,3-, 2,4-, 2,5-, 2,6-, 3,4-, and 3,5-dichloronitrobenzenes; the various trichloronitrobenzenes; the corresponding fluoro, bromo and iodo compounds; the various dimethyl-, diethyl-, and dibutylnitrobenzenes, nitrobiphenyls, benzylnitrobenzenes, nitronaphthalenes, di- and trinitrobenzenes, nitro-N,N-diethylanilines, nitrodiphenyl esters, nitro-N-ethylacetanilides, nitrobenzylcyanides, nitrophenyl acetates, nitropyridine-N-oxides, nitroquinolines, nitroisoquinolines, nitrothiophenes, and the like bearing one or more ar-chloro, fluoro, bromo, or iodo substituents and containing at least one replaceable hydrogen in an appropriate position. Since there is a tendency for iodo substituents to be removed under the conditions of the substitution reaction of the invention, the preferred halonitroaromatic compounds are those in which the halo substituents are fluoro, chloro, or bromo, most preferably fluoro or chloro.

In some cases, polynitroaromatic reactants may undergo substitution reactions whereby one of the nitro groups is replaced by the ester reactant. Therefore, the possibility of this competitive reaction should be kept in mind when selecting a polynitroaromatic for use in the process.

In each of the aforementioned embodiments of the invention, the preferred nitroaromatic compounds are nitrobenzenes having a replaceable hydrogen in the position para to the nitro group, since the nucleophilic substitution reaction of the invention tends to be highly selective on the para position, and the use of such compounds therefore leads to the production of nitrobenzeneacetic acid esters which are ideally suited for the synthesis of anti-inflammatory agents of the type described in the aforementioned references. Even more preferred in the case of the halonitrobenzenes are such nitrobenzenes having a halo substituent in a position ortho to the nitro group. Halonitrobenzenes which are especially preferred at 2-chloronitrobenzene and 2-fluoronitrobenzene, which are readily converted with high selectivity into such products as 2-[3-chloro-4-(3-pyrrolinyl)phenyl]propionic acid, 2-(2-fluoro-4-biphenylyl)propionic acid and related anti-inflammatory agents. A non-halogenated nitroaromatic compound that is particularly preferred is nitrobenzene, which is readily converted with high selectivity into pharmaceutically-active agents such as 2-[4-(1,3-dihydro-1-oxo-2H-isoindol-2-yl)phenyl]propionic acid, 2-[4-(1,3-dihydro-1-oxo-2H-isoindol-2-yl)phenyl]butyric acid, and analogs thereof.

The alpha,alpha-disubstituted acetic acid esters that can be used in the practice of the invention also include a variety of such compounds, which—in general—may be represented by the formula:

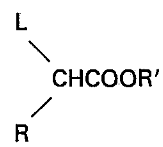

wherein L is a leaving group; R is halo (preferably chloro) or more preferably a hydrocarbyl (e.g., alkyl, alkenyl, alkynyl, cycloalkyl, cycloalkenyl, aryl, aralkyl, etc.) or hydrocarbyloxy hydrocarbyl (e.g., alkoxyalkyl, aryloxyalkyl, alkoxyaryl, alkoxy cycloalkyl, etc.) group which most preferably contains up to about 10 carbons; and R′ is a hydrocarbyl group which preferably contains up to about 10 carbons and most preferably is an alkyl group.

Exemplary leaving groups, L, include halo, aryloxy, haloaryloxy, alkylthio, cycloalkylthio, arylthio, aralkylthio, haloalkylthio, halocycloalkylthio, haloarylthio, haloaralkylthio, or—less preferably—alkoxy, cycloalkoxy, aralkoxy, haloalkoxy, halocycloalkoxy, haloaralkoxy, and the like, as well as other suitable leaving groups which have been described in the literature, e.g., in Golinski et al., "Vicarious' Nucleophilic Substitution of Hydrogen in Aromatic Nitro Compounds, *Tetrahedron Letters*, Vol. 37, pp. 3495—8 (1978) and in Makosza et al., "Vicarious Substitution of Hydrogen in Aromatic Nitro Compounds with Acetonitrile Derivatives", *Journal of Organic Chemistry*, Vol. 45, pp. 1534—5 (1980).

When the leaving group is an organic group, it is generally preferred that the group—which does not remain in the product—contain not more than about 10 carbons, although organic leaving groups having an even higher carbon content are satisfactory in the practice of the invention. Preferably, the leaving group is halo, i.e., chloro, bromo, fluoro, or iodo; and it is more preferably chloro or bromo, most preferably chloro.

A few examples of utilizable alpha,alpha-disubstituted acetic acid esters are alpha-chloropropionates such as methyl, ethyl, propyl, isopropyl, butyl, *t*-butyl, cyclohexyl, phenyl, and benzyl 2-chloropropionates; the corresponding alpha-bromopropionates; and other alpha-substituted monocarboxylic acid esters such as methyl 2-methylmercaptopropionate; ethyl 2-butylmercapto propionate, methyl 2-phenoxybutyrate, phenyl 2-methylmercapto propionate, butyl 2-cyclohexylmercaptovalerate, methyl 2-(4-fluorophenoxy)propionate, and the like. The alpha-haloalpha-hydrocarbylacetic acid esters, i.e., esters of alpha-halomonocarboxylic acids containing at least three carbons, are especially preferred, although similar esters in which the alpha-halo substituent is replaced by one of the other leaving groups mentioned above are also highly desirable.

In another highly desirable embodiment of the invention, the alpha,alpha-disubstituted acetic acid ester is an alpha,alpha-dihaloacetic acid ester, most preferably an alpha,alpha-dichloroacetic acid ester, which leads to the formation of a product having a reactive halo substituent in the alpha-position, e.g., a product corresponding to the formula:

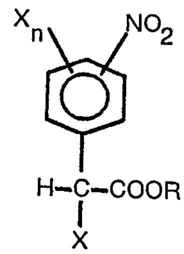

wherein X is halo, preferably chloro; R is a hydrocarbyl group, preferably containing up to about 10 carbons; and n is an integer of 1 to 3. Such products enable facile synthesis of a variety of end products. Most preferably the nitro group is in the position para to the ester substituent, although it may be located in an ortho position.

Illustrative aprotic solvents which may be employed in the process of the invention include ethers such as diethyl ether, dibutyl ether, 1-ethoxyhexane, tetrahydrofuran, 1,4-dioxane, 1,3-dioxolane, diglyme, 1,2-diethoxyethane, anisole, etc.; tertiary amines such as pyridine, N-ethylpiperidine, triethyl amine, tributyl amine, N,N-diphenyl-N-methyl amine, N,N-dimethylaniline, etc.; and other aprotic solvents. However, the preferred aprotic solvents are dipolar aprotic solvents such as dimethyl sulfoxide, N,N-dimethylformamide, N,N-dimethylacetamide, dimethyl sulfone, tetramethylene sulfone, N-methylpyrrolidone, and the like.

Bases useful in the practice of the invention include all bases strong enough to activate the ester reactant, e.g., alkaline earth metal compounds such as calcium oxide, calcium hydride, calcium hydroxide, barium oxide, barium hydroxide, magnesium hydroxide, zinc hydroxide, etc. However, the base is preferably an alkali metal compound, e.g., an organoalkali metal compound, alkali metal hydride, alkali metal hydroxide, alkali metal oxide, alkali metal amide, or alkali metal alcoholate, such as butyllithium, phenyllithium, ethylsodium, amylsodium butylpotassium, benzylpotassium, sodium dimsylate (i.e., the sodium salt of diethylsulfoxide), sodium hydride, potassium hydride, sodium hydroxide, potassium hydroxide, sodium oxide, potassium oxide, sodium amide, potassium amide, lithium diisopropylamide, sodium methoxide, potassium t-butoxide, the sodium salt of the monomethylether of ethylene glycol, sodium phenoxide, and the like. Ordinarily the use of sodium hydride or potassium hydride will be found most convenient and economical.

Use of an alkali metal compound as the base permits the alternatives of using the alkali metal compound alone or in conjunction with a phase transfer catalyst, such as a quaternary ammonium salt, ethylene glycol, or a suitable crown ether. When a phase transfer catalyst is employed (1) the alkali metal compound may be any of the alkali metal compounds generically or specifically indicated above, although the type of alkali metal compound being used determines the type of crown ether that is preferably utilized—lithium bases generally calling for the use of a 12-crown-4 ether, sodium bases generally calling for the use of a 15-crown-5 ether, and potassium bases generally calling for the use of an 18-crown-6 ether, and (2) the reaction medium may be any of the aprotic solvents mentioned above, or it may be an inert liquid hydrocarbon such as benzene, toluene, xylene, hexane, heptane, isooctane, or the like.

When an alkali metal hydride, especially a highly pure alkali metal hydride, is employed as the base, it is desirable to include a small amount of a transfer agent such as water, alcohol, or the like in the system. It is believed that the transfer agent activates the hydride by reacting therewith to form a small amount of the alkali metal hydroxide or alcoholate.

The nitroarylacetic acid ester synthesis of the invention is conducted in a substantially anhydrous reaction system, and accordingly, except when a small amount of water (which is itself consumed by reaction with the alkali metal hydride), is employed as a transfer agent, the components of the reaction system should be brought together and maintained under a dry inert atmosphere. Thus, while it is possible to conduct the process in the presence of air, it is desirable to maintain the reaction system under an atmosphere of dry nitrogen or the like. Since the reaction itself is normally an exothermic reaction, with its initiation readily ascertainable by noting the exotherm produced, the reactants are ordinarily brought together at ambient temperatures, although the temperature may be raised or lowered to suit the needs of the occasion if desired.

The nitroaromatic compound and alpha,alpha-disubstituted acetic acid ester may be used in amounts such as to provide a stoichiometric excess of either of the reactants or the stoichiometric amount of each. However, when a stoichiometric excess of the nitroaromatic compound is employed, the quantity of product obtainable will be limited by the quantity of ester used, so it is desirable to utilize a stoichiometric excess of the ester. The amount of base employed is preferably such as to provide at least two molar equivalents of base per mol of nitroaromatic compound, since the use of smaller amounts—although permitting the reaction to occur—makes the base the limiting reagent.

The mode of addition of the ingredients of the reaction system is not particularly critical. Accordingly, it is convenient to add the nitroaromatic compound to a mixture of the other materials, add the base to a mixture of the other materials, add the reactants to a mixture of the base and aprotic solvent, introduce all four ingredients simultaneously into the reaction zone, or the like. Since the reaction ordinarily proceeds very rapidly, long reaction times are not required. The reaction will usually be completed within a matter of minutes or a few hours at ambient temperatures.

When derivatives of the nitroarylacetic acid esters are desired, they may be prepared by employing conventional techniques to convert to the desired derivatives the nitroarylacetic acid esters made in accordance with the present invention. Thus, for example:

(A) an alkyl 2-(4-nitrobenzene)propionate or an alkyl 2-(3-chloro-4-nitrobenzene)propionate synthesized by the process of this invention may be hydrogenated to an alkyl 2-(4-aminobenzene)-propionate, which in turn may be reacted with phthalic anhydride to form an alkyl 2-(4-phthalimidophenyl)-propionate, which may be reduced and hydrolyzed to 2-[4-(1,3-dihydro-1-oxo-2H-isoindol-2-yl)-phenyl]-propionic acid,

(B) an alkyl 2-(3-chloro-4-nitrobenzene)propionate synthesized by the process of this invention may be hydrolyzed to 2-(3-chloro-4-nitrobenzene)propionic acid, which in turn may be reduced to 2-(4-aminobenzene)propionic acid, reacted with phthalic anhydride to form 2-(4-phthalimidophenyl)-propionic acid and reduced to 2-[4-(1,3-dihydro-1-oxo-2H-isoindol-2-yl)-phenyl]propionic acid, and

(C) an alkyl 2-(3-fluoro-4-nitrobenzene)propionate synthesized by the process of this invention may be selectively hydrogenated to an alkyl 2-(4-amino-3-fluorobenzene)propionate, which in turn may be subjected to a Gomberg-Bachmann reaction to replace the amino group with a phenyl group and form an alkyl 2-(2-fluoro-4-biphenylyl)propionate, which may be hydrolyzed to 2-(2-fluoro-4-biphenylyl)propionic acid.

The particular conventional techniques used to convert the nitroarylacetic acid esters into their various derivatives are not critical. It may sometimes be desirable to use certain particular techniques for the preparation of the derivatives, e.g. the techniques taught in the aforementioned Carney et al. and Carney et al. II references. However, the overall processes for preparing the derivatives are simplified and made more efficient and economical by the present simplication of the synthesis of the nitroarylacetic acid esters, regardless of the particular techniques used to convert them into their various derivatives.

The invention includes the preparation of pharmaceutical or veterinary formulations comprising those of said derivatives which have therapeutic activity, by in each case making said derivatives as above and formulating them for pharmaceutical or veterinary use, respectively.

As indicated the present invention is particularly advantageous in providing a readier and more economical route to the synthesis of pharmaceuticals and other chemical products that can be prepared from nitroarylacetic acid esters. Such products include, not only those mentioned above, but a variety of products, such as products disclosed in U.S. Patents 3,641,040, 3,657,230, 3,767,805, 3,868,391, 3,936,467, 3,993,763, 3,997,669, 4,010,274, 4,118,504, 4,126,691, 4,163,788 and 4,239,901.

The following Examples are given to illustrate the invention and are not intended as a limitation thereof.

Synthesis of nitroarylacetic acid esters

Example 1

A flame-dried flask was charged with 3.0 ml (26 mmols) of 2-chloronitrobenzene, 2.9 ml (26 mmols) of methyl 2-chloropropionate, and 25 ml of N,N-dimethylformamide that had previously been dried over 3-Angstrom molecular sieves. The flask was supported in a water bath, and 1.5 g (31 mmols) of sodium hydride were incrementally added over a period of 20 minutes as a 50% slurry in hydrocarbon oil. Upon the addition of the first incremental portion of the sodium hydride, the reaction mixture became red and thereafter turned deep purple as more of the base was added. After all of the sodium hydride had been charged to the flask, the mixture was stirred at room temperature under a nitrogen atmosphere for 18 hours. The reaction mixture was then poured into 1N HCl and extracted with portions of diethyl ether to form ether layers which were combined, extracted with water, dried over $MgSO_4$, and stripped to give a black oil. An aliquot of the oil was analyzed by means of a gas chromatograph coupled with a mass spectrometer (GC/MS) and found to contain a product having the empirical formula $C_{10}H_{10}ClNO_4$. Another aliquot was subjected to preparative thin layer chromatography, and NMR analysis of the isolated product was consistent with the compound methyl 2-(3-chloro-4-nitrobenzene)propionate.

Example 2

A flame-dried flask was purged with nitrogen to provide an inert atmosphere and charged with 5.1 g (110 mmols) of a 50% dispersion of sodium hydride in hydrocarbon oil. The sodium hydride was washed with 15 ml of petroleum ether (b.p. 35—60°C.), after which the flask was charged with 15 ml of N,N-dimethylformamide that had been dried over 3-Angstrom molecular sieves. Then a reaction mixture was formed by the dropwise addition, over 20 minutes, of a solution of 6 ml (51 mmols) of 2-chloronitrobenzene and 6 ml (53 mmols) of methyl 2-chloropropionate in 10 ml of N,N-dimethylformamide. The mixture turned purple and became hot during the dropwise addition. After completion of this addition, the reaction mixture was stirred for an additional 15 minutes, poured into 200 ml of 1N HCl,

and extracted with three 150 ml portions of diethyl ether. The ether layers were combined, dried over $MgSO_4$, and concentrated to give a black oil which was chromatographed on a 200 g silica gel column eluted with 30% methylene chloride/70% petroleum ether. The appropriate fractions were combined and concentrated to afford 8.2 g of material which contained 94% (by GC area percent), i.e., 7.7 g, of methyl 2-(3-chloro-4-nitrobenzene)propionate—a yield of 62%.

Example 3

Example 2 was repeated except that the 2-chloronitrobenzene/2-chloropropionate/N,N-dimethylformamide solution was added over a period of 30 minutes during which the temperature of the reaction mixture was maintained at 25—30°C. by intermittent cooling with an ice/water bath, and stirring of the reaction mixture was continued for 30 minutes after completion of this dropwise addition. The reaction mixture was then poured into 150 ml of 1N HCl and extracted with four 150 ml portions of diethyl ether. The ether layers were combined, dried over $MgSO_4$, and concentrated to give an oil which was adsorbed on 15 g of silica gel (230—400 mesh). This was loaded on a column of 200 g of silica which was eluted with 30% dichloromethane/70% petroleum ether under nitrogen pressure to give 0.65 g of unreacted 2-chloronitrobenzene and 8.4 g of methyl 2-(3-chloro-4-nitrobenzene)propionate—a yield of about 68%.

Example 4

Into a flame-dried flask under a blanket of nitrogen were placed 300 mg of a 50% dispersion of sodium hydride in hydrocarbon oil (6.3 mmols) and 5 ml of N,N-dimethylformamide. The flask was then charged with a solution of 0.56 ml (4.8 mmols) of 2-chloronitrobenzene and 0.65 ml (5.0 mmols) of ethyl 2-bromopropionate in 2 ml of N,N-dimethylformamide, which was added dropwise over a period of 5 minutes. Upon the addition of the first drop of this solution, the mixture became orange-red in color; and about 5 minutes after the dropwise addition had been completed, the mixture turned deep purple, and a pronounced exotherm was noted. Then a second portion of sodium hydride (4.2 mmols) was added, and another exotherm was observed. A small portion of the reaction mixture was then partitioned between 1N HCl and diethyl ether. Analysis of the ether layer by GC/MS indicated that ethyl 2-(3-chloro-4-nitrobenzene)propionate had been formed.

Example 5

A flame-dried flask was purged with nitrogen to provide an inert atmosphere and charged with 75 mg (1.9 mmols) of a 60% dispersion of sodium hydride in mineral oil. This was washed with three 2 ml portions of petroleum ether (b.p. 35—60°C.) and slurried in 1.0 ml of N,N-dimethylformamide. A solution of 103 mg (0.65 mmol) of 2-chloronitrobenzene and 126 mg (0.65 mmol) of ethyl 2-phenoxypropionate in 1.0 ml of N,N-dimethylformamide was added dropwise to the sodium hydride slurry, and the resulting purple mixture was stirred for 45 minutes, after which it was poured into 20 ml of 1N HCl. The aqueous mixture was extracted with three 20 ml portions of diethyl ether, and the ether layers were combined, dried over $MgSO_4$, concentrated, and placed in a 2 mm silica gel thin layer chromatographic (TLC) plate. Elution of the plate with 50% petroleum ether/50% dichloromethane afforded 4.6 mg of 2-chloronitrobenzene, 15 mg of ethyl 2-phenoxypropionate, and 53 mg of ethyl 2-(3-chloro-4-nitrobenzene)propionate.

Example 6

Into a flame-dried flask under nitrogen were placed 50 mg (1.25 mmols) of a 60% dispersion of sodium hydride in mineral oil, which was washed with three 2 ml portions of petroleum ether (b.p. 35—60°C) and slurried in 1.0 ml of N,N-dimethylformamide. Then a solution of 100 mg (0.635 mmol) of 4-chloronitrobenzene and 83 mg (0.677 mmol) of methyl 2-chloropropionate in 1.0 ml of N,N-dimethylformamide was added dropwise to the slurry, and the resulting purple mixture was stirred for 15 minutes and poured into 20 ml of 1N HCl. The aqueous mixture was extracted with three 20 ml portions of diethyl ether, and the ether layers were combined, dried over $MgSO_4$, concentrated, and placed on a 2 mm silica gel TLC plate. Elution of the plate with 50% petroleum ether/50% dichloromethane afforded 43 mg of 4-chloronitrobenzene and 13 mg of methyl 2-(2-nitro-5-chlorobenzene)propionate.

Example 7

Into a flame-dried flask under nitrogen were placed 140 mg (1.24 mmols) of potassium t-butoxide, 23 mg (0.064 mmol) of dibenzo 18-crown-6 ether, and 1.0 ml of toluene. While this mixture was vigorously stirred in a room temperature water bath, a solution of 106 mg (0.670 mmol) of 2-chloronitrobenzene and 124 mg (0.633 mmol) of ethyl 2-bromobutyrate in 1.0 ml of toluene was added dropwise. The resulting purple mixture was stirred for 5 minutes and poured into 20 ml of 1N HCl. Then the aqueous mixture was extracted with three 20 ml portions of diethyl ether, and the ether layers were combined, dried over $MgSO_4$, concentrated, and placed on a 2 mm silica gel TLC plate. Elution of the plate with 60% petroleum ether/40% dichloromethane afforded 45 mg of 2-chloronitrobenzene and 27 mg of ethyl 2-(3-chloro-4-nitrobenzene)-butyrate.

Example 8

A flame-dried flask was purged with nitrogen to provide an inert atmosphere and charged with 2.8 g

(58 mmols) of a 50% dispersion of sodium hydride in mineral oil. This was washed with three 10 ml portions of petroleum ether (b.p. 35—60°C.), dried in a nitrogen stream, slurried in 20 ml of N,N-dimethylformamide, and cooled in an ice/water bath. A solution of 3.7 g (26 mmols) of 2-fluoronitrobenzene and 3.6 g (29 mmols) of methyl 2-chloropropionate in 5 ml of N,N-dimethylformamide was added dropwise to the vigorously stirred sodium hydride slurry over a period of 15 minutes. After the addition had been completed, the cooling bath was removed but was reapplied periodically to keep the temperature of the reaction mixture below 30°C. while the mixture was stirred for an additional 30 minutes. The reaction mixture was then poured into 150 ml of cold 1N HCl, and the resulting aqueous mixture was extracted with three 100 ml portions of diethyl ether. The ether layers were combined, dried over $MgSO_4$, and concentrated to give 7.8 g of an orange oil. Chromatography of this oil on a column of 150 g of 230—400 mesh silica gel afforded 3.1 g of a fraction (eluted with 40% dichloromethane/60% petroleum ether) containing methyl 2-(3-fluoro-4-nitrobenzene)propionate.

Example 9

Into a flame dried flask fitted with a mechanical stirrer, under nitrogen, were placed 3.6 g of 60% NaH in mineral oil (0.09 mol). This was washed with three 15 ml portions of petroleum ether (b.p. 35—60°C.), dried in a nitrogen stream, and slurried in 25 ml of N,N-dimethylformamide (stored over 3 Angstrom molecular sieves). Then ten drops of a solution of 5.0 g of nitrobenzene (0.041 mol) and 5.5 g of methyl 2-chloropropionate (0.045 mol) in 5 ml of N,N-dimethylformamide were added to the NaH slurry. After about 3 minutes the mixture became deep purple in color and an exotherm was noted, indicating the reaction had begun. The rest of the reactant solution was added dropwise to the NaH slurry over 22 minutes, and an ice-water bath was periodically applied to the flask so that the temperature remained between 20 and 30°C. The mixture was allowed to stir for 15 minutes at 20—30°C. after the addition was complete and was then poured into 200 ml of 1N HCl. This aqueous mixture was extracted with three 150 ml portions of diethyl ether. The ether layers were combined, dried (magnesium sulfate), and concentrated to give 10 g of brown oil. Gas chromatographic analysis of this oil indicated it contained 6.9% nitrobenzene and 54% methyl 2-(4-nitrobenzene)propionate. From the oil was distilled 4.6 g of 96% methyl 2-(4-nitrobenzene)propionate (0.5 mm, 125°C.).

Example 10

A reaction was carried out as described in Example 9 but was worked up as follows: The reaction mixture was poured into 200 ml of water and the resulting aqueous mixture (pH 12.9) was successively extracted with three 150 ml portions of petroleum ether (b.p. 35—60°C.), three 150 ml portions of toluene, three 150 ml portions of diethyl ether, and three 150 ml portions of dichloromethane. Gas chromatographic analyses indicated that essentially all of the methyl 2-(4-nitrobenzene)propionate was located in the petroleum ether extracts. These were combined, dried (magnesium sulfate) and concentrated to give 3.7 g of an oil containing 59% methyl 2-(4-nitrobenzene)propionate. From the oil was distilled 2.0 g of 94% methyl 2-(4-nitrobenzene)propionate (0.5 mm, 125°C.).

Example 11

Another reaction was carried out as in Example 9 except that the workup was as follows: The reaction mixture was poured into 200 ml of water and 96% sulfuric acid was added until the pH was 6.9. The resulting aqueous mixture was successively extracted-with three 150 ml portions of petroleum ether (b.p. 35—60°C.), three 150 ml portions of toluene, three 150 ml portions of diethylether and three 150 ml portions of dichloromethane. Gas chromatographic analyses indicated that essentially all of the methyl 2-(4-nitrobenzene)propionate was contained in the petroleum ether extracts. These were combined, dried (magnesium sulfate), and concentrated to give 4.4 g of an oil containing 71% methyl 2-(4-nitrobenzene)-propionate.

Example 12

A reaction mixture formed as in Example 9 was worked up as follows: The reaction mixture was poured into 200 ml of water and 96% sulfuric acid was added until the pH was 2.9. The resulting aqueous mixture was then successively extracted with three 150 ml portions of petroleum ether (b.p. 35—60°C.), three 150 ml portions of toluene, three 150 ml portions of diethyl ether, and three 150 ml portions of dichloromethane. Gas chromatographic analyses indicated that most of the methyl 2-(4-nitrobenzene)-propionate was in the petroleum ether extracts although some was contained in the toluene extracts. The petroleum ether layers were combined, dried with magnesium sulfate, and concentrated to give 4.8 g of an oil containing 72% methyl 2-(4-nitrobenzene)propionate. The toluene extracts were combined, dried with magnesium sulfate, and concentrated to give 3.5 g of an oil containing 5.0% methyl 2-(4-nitrobenzene)-propionate.

Example 13

The following workup procedure was applied to another reaction mixture formed as in Example 9. The reaction mixture was poured into a solution of 1.25 ml of 96% sulfuric acid in 200 ml of water. About 3 ml of a black oil settled to the bottom of the aqueous mixture and was removed. The oil was triturated with three

10 ml portions of petroleum ether (b.p. 35—60°C.), and the petroleum ether layers were combined and dried over magnesium sulfate. The dried layers were concentrated to give 1.3 g of oil (fraction 1) containing 72% methyl 2-(4-nitrobenzene)propionate (by gas chromatographic analysis). The aqueous mixture was extracted with three 150 ml portions of petroleum ether and the organic layers were combined, dried (magnesium sulfate) and concentrated to give 2.7 g of an oil (fraction 2) containing 77% methyl 2-(4-nitrobenzene)propionate. Then the black oil that had separated from the water was recombined with the water layer and the resulting mixture was extracted with three 150 ml portions of diethyl ether. The ether layers were combined, dried (magnesium sulfate) and concentrated to give 5.3 g of an oil (fraction 3) containing 39% methyl 2-(4-nitrobenzene)propionate.

Example 14

Into a flame dried 1 liter flask fitted with a mechanical stirrer, under nitrogen, were placed 18.0 g of 60% NaH in mineral oil (0.450 mol) and 100 ml of N,N-dimethylformamide (stored over 3 Angstrom molecular sieves). To the resulting slurry were added 50 drops of a solution of 25.0 g of nitrobenzene (0.203 mol) and 27.5 g of methyl 2-chloropropionate (0.224 mol) in 25 ml of N,N-dimethylformamide. After about 6 minutes the mixture became deep purple in color and an exotherm was noted, indicating the reaction had begun. The rest of the reactant solution was added dropwise to the NaH slurry over 30 minutes and an ice-water bath was periodically applied to the flask so that the temperature remained between 20 and 30°C. The mixture was allowed to stir for 20 minutes at 20—30°C. after the addition was complete and was then poured into 1 liter of 1N HCl. This aqueous mixture was extracted with three 500 ml portions of dichloromethane. The organic layers were combined, dried (magnesium sulfate), and concentrated to give 142 g of black liquid. From this liquid was distilled 79.1 g of N,N-dimethylformamide (fraction 1, 10 mm, 30—70°C.), followed by 34.6 g of orange oil (fraction 2, 10 mm, 65—180°C.) which contained 7.0% nitrobenzene (by gas chromatographic analysis), 75% methyl 2-(4-nitrobenzene)propionate (by gas chromatographic analysis) and some mineral oil (observed as an insoluble upper layer).

Synthesis of derivatives

Example 15

Into 20 ml of 5N NaOH were placed 20 g of fraction 2 obtained in Example 14, i.e., 15 g (72 mmols) of methyl 2-(4-nitrobenzene)propionate, and the resulting mixture was stirred vigorously under nitrogen and cooled in a 25°C. water bath. The mixture became homogeneous after 20 minutes. After 90 minutes the mixture was extracted with three 30 ml portions of dichloromethane and the aqueous layer was stirred at 50°C. under aspirator vacuum to remove residual dichloromethane. The aqueous solution was cooled in an ice-water bath and 37% HCl was added dropwise to a pH of 7. The cooling bath was removed, 200 mg Calgon CPG decolorizing carbon (−70 mesh) was added, the mixture was stirred for 10 minutes, and the carbon was removed by filtration through Celite. The filtrate was cooled in an ice-water bath and 37% HCl was added to a pH of 1. The precipitated solid was removed by filtration, washed with 25 ml of cold 1N HCl, and dried at 1 mm for 19 hours over phosphorus pentoxide to give 13.3 g of 2-(4-nitrobenzene)propionic acid (mp 83—85°C.).

Example 16

Fraction 2 from Example 13 (2.7 g of oil containing 77% methyl 2-(4-nitrobenzene)propionate was added to 5 ml of 5N NaOH and the resulting heterogeneous mixture was stirred vigorously under nitrogen for 1.5 hours. The mixture became homogeneous during this time. It was extracted with three 10 ml portions of dichloromethane and the organic layers were combined, dried with magnesium sulfate, and concentrated to give 0.37 g of an oil containing 88% nitrobenzene and 2% methyl 2-(4-nitrobenzene)-propionate (by gas chromatographic analysis). The aqueous layer was cooled with an ice-water bath and acidified with 37% HCl. The solid which precipitated was removed by filtration and dried under high vacuum overnight to give 1.1 g of crude 2-(4-nitrobenzene)propionic acid (mp 64—68°C.).

Example 17

A mixture of 1.0 g (3.7 mmols) of methyl 2-(3-chloro-4-nitrobenzene)propionate (91% pure by GC), 0.37 g (4.5 mmols) of anhydrous sodium acetate, and 0.1 g of 7% palladium on carbon in 15 ml of methanol was hydrogenated in an agitated vessel at 50 psig for 1.6 hours. The mixture was then filtered, and the catalyst was washed with two 5 ml portions of methanol. The filtrate was concentrated under vacuum, and the residue was partitioned between 20 ml of dichloromethane and 20 ml of saturated sodium bicarbonate solution. The organic layer was dried over $MgSO_4$ and concentrated to give 0.66 g of an oil which, by GC, contained 89% methyl 2-(4-aminobenzene)propionate.

This invention is susceptible to considerable variation within the scope thereof.

**Claims**

1. A process for the preparation of a nitroarylacetic acid ester which comprises reacting a nitroaromatic compound with an alpha,alpha-disubstituted acetic acid ester in a substantially anhydrous aprotic solvent and in the presence of a base.

2. A process as claimed in claim 1, wherein the nitroaromatic compound is devoid of halogen on the aromatic ring carrying the nitro group.

3. A process as claimed in claim 1, wherein the nitroaromatic compound is a halonitroaromatic compound having a halo substituent on the aromatic ring carrying the nitro group.

4. A process as claimed in claim 2 or claim 3, wherein the nitroaromatic compound is a mononitrobenzene having an unsubstituted position para to the nitro group.

5. A process as claimed in claim 4, wherein the mononitrobenzene is nitrobenzene or an o-halonitrobenzene.

6. A process as claimed in claim 1, wherein the nitroaromatic compound is a chloronitrobenzene, preferably o-chloronitrobenzene.

7. A process as claimed in claim 1, wherein the nitroaromatic compound is a fluoronitroaromatic compound, preferably o-fluoronitrobenzene.

8. A process as claimed in claim 1, wherein the nitroaromatic compound is a halonitroaromatic compound wherein the halo substituent has an atomic number of at least 17, the resulting nitroarylacetic acid ester being a halonitroarylacetic acid ester.

9. A process as claimed in any one of claims 1 to 8, wherein the alpha,alpha-disubstituted acetic acid ester is an alpha-halomonocarbocylic acid ester containing at least three carbons in the acid moiety.

10. A process as claimed in any one of claims 1 to 8 wherein the alpha,alpha-disubstituted acetic acid ester is an alkyl alpha-chloro- or alpha-bromopropionate.

11. A process as claimed in any one of claims 1 to 10, wherein the solvent is a dipolar aprotic solvent.

12. A process as claimed in any one of claims 1 to 11, wherein the base is an alkali metal compound, preferably sodium hydride or potassium hydride.

13. A process as claimed in any one of claims 1 to 12, wherein the nitroarylacetic acid ester is a 2-(4-nitrobenzene)acetic acid ester or an alkyl 2-(4-nitrobenzene)propionate.

14. A process as claimed in claim 13, wherein the nitroarylacetic acid ester is an alkyl 2-(3-halo-4-nitrobenzene)propionate.

15. A process for the preparation of an alkyl 2-(4-aminobenzene)propionate, which process comprises effecting the steps of a process as claimed in claim 13 to produce an alkyl 2-(4-nitrobenzene)propionate or an alkyl 2-(3-chloro-4-nitrobenzene)propionate and subjecting the resulting product to hydrogenation to form an alkyl 2-(4-aminobenzene)propionate.

16. A process for preparing an alkyl 2-(4-phthalimidophenyl)propionate comprising effecting the steps of a process as claimed in claim 15 and reacting the resulting alkyl 2-(4-aminobenzene)propionate with phthalic anhydride.

17. A process for preparing 2-[4-(1-,3-dihydro-1-oxo-2H-isoindol-2-yl)phenyl]propionic acid comprising effecting the steps of a process as claimed in claim 16 and subjecting the resulting alkyl 2-(4-phthalimidophenyl)propionate to reduction and hydrolysis.

18. A process for the preparation of a 2-(4-nitrobenzene)propionic acid comprising effecting the steps of a process as claimed in claim 13 to produce an alkyl 2-(4-nitrobenzene)propionate and hydrolysing the resulting product.

19. A process as claimed in claim 18, wherein either the product acid is 2-(4-nitrobenzene)propionic acid or the alkyl 2-(4-nitrobenzene)propionate is an alkyl 2-(3-chloro-4-nitrobenzene)propionate and the product acid is 2-(3-chloro-4-nitrobenzene)propionic acid.

20. A process for preparing 2-(aminobenzene)propionic acid comprising effecting the steps of a process as claimed in claim 19 to produce 2-(4-nitrobenzene)propionic acid or 2-(3-chloro-4-nitrobenzene)-propionic acid and reducing the resulting acid.

21. A process for preparing 2-(4-phthalimidophenyl)propionic acid comprising effecting the steps of a process as claimed in claim 20 and reacting the resulting 2-(4-aminobenzene)propionic acid with phthalic anhydride.

22. A process for preparing 2-[4-(1,3-dihydro-1-oxo-2H-isoindol-2-yl)phenyl]propionic acid comprising effecting the steps of a process as claimed in claim 21 and reducing the resulting 2-(4-phthalimidophenyl)-propionic acid.

23. A process for the preparation of an alkyl 2-(4-amino-3-chloro or 3-fluoro benzene)propionate comprising effecting the steps of a process as claimed in claim 14 to produce an alkyl 2-(3-chloro or 3-fluoro-4-nitrobenzene) propionate and selectively hydrogenating the resulting product.

24. A process for preparing an alkyl 2-[3-chloro-4-(3-pyrrolinyl)phenyl]propionate comprising effecting the steps of a process as claimed in claim 23 to produce an alkyl 2-(4-amino-3-chlorobenzene)propionate and reacting the resulting product with a 1,4-dihalo-2-butene.

25. A process for preparing 2-[3-chloro-4-(3-pyrrolinyl)phenyl]propionic acid comprising effecting the steps of a process as claimed in claim 24 and hydrolysing the resulting alkyl 2-[3-chloro-4-(3-pyrrolinyl)phenyl]propionate.

26. A process for preparing an alkyl 2-(2-fluoro-4-biphenylyl)propionate comprising effecting the steps of process as claimed in claim 23 to produce an alkyl 2-(4-amino-3-fluorobenzene)propionate and subjecting the resulting product to a Gomberg-Bachmann reaction to replace the amino group with a phenyl group.

27. A process for preparing 2-(2-fluoro-4-biphenylyl)propionic acid comprising effecting the steps of a process as claimed in claim 26 and hydrolysing the resulting alkyl 2-(2-fluoro-4-biphenylyl)propionate.

28. A method for making a pharmaceutical or veterinary formulation comprising preparing 2-(2-fluoro-4-biphenylyl)propionic acid or 2-[3-chloro-4-(3-pyrrolinyl)phenyl]propionic acid or 2-[4-(1,3-dihydro-1-oxo-2H-isoindol-2-yl)phenyl]propionic acid by a process as claimed in claim 27, claim 25, or either of claims 17 and 22, respectively, and formulating the resulting acid for pharmaceutical or veterinary use, respectively.

**Patentansprüche**

1. Verfahren zur Herstellung eines Nitroarylessigsäureesters, in dem man eine nitroaromatische Verbindung mit einem α,α-disubstituierten Essigsäureester in einem, im wesentlichen wasserfreien aprotischen Lösungsmittel und in Gegenwart einer Base, zur Umsetzung bringt.

2. Verfahren nach Anspruch 1, in dem die nitroaromatische Verbindung kein halogen im aromatischen Ring aufweist, der die Nitrogruppe trägt.

3. Verfahren nach Anspruch 1, in dem die nitroaromatische Verbindung eine halonitroaromatische Verbindung ist, die einen Halosubstituenten im aromatischen Ring aufweist, der die Nitrogruppe trägt.

4. Verfahren nach Anspruch 2 oder 3, in dem die nitroaromatische Verbindung Mononitrobenzol ist, das in der para-Stellung zur Nitrogruppe unsubstituiert ist.

5. Verfahren nach Anspruch 4, in dem das Mononitrobenzol Nitrobenzol oder ein o-Halonitrobenzol ist.

6. Verfahren nach Anspruch 1, in dem die nitroaromatische Verbindung Chlornitrobenzol, vorzugsweise o-Chlornitrobenzol ist.

7. Verfahren nach Anspruch 1, in dem die nitroaromatische Verbindung eine fluornitroaromatische Verbindung, vorzugsweise o-Fluornitrobenzol ist.

8. Verfahren nach Anspruch 1, in dem die nitroaromatische Verbindung eine halonitroaromatische Verbindung ist, in der der Halosubstituent eine Atomzahl von mindestens 17 hat und der gebildete Nitroarylessigsäureester ein Halonitroarylessigsäureester ist.

9. Verfahren nach Anspruch 1 bis 8, in dem der α,α-disubstituierte Essigsäureester ein α-Halomonokarbonsäureester ist, der mindestens drei C-Atome im Säureanteil besitzt.

10. Verfahren nach Anspruch 1 bis 8, in dem der α,α-disubstituierte Essigsäureester ein Alkyl -α-chlor- oder α-Brompropionat ist.

11. Verfahren nach Anspruch 1 bis 10, in dem das Lösungsmittel ein dipolares aprotisches Lösungsmittel ist.

12. Verfahren nach Anspruch 1 bis 11, in dem die Base eine Alkalimetallverbindung, vorzugsweise Natriumhydrid oder Kaliumhydrid ist.

13. Verfahren nach Anspruch 1 bis 12, in dem der Nitroarylessigsäureester ein 2-(4-Nitrobenzol)essigsäureester oder ein Alkyl-2-(4-nitrobenzol)propionat ist.

14. Verfahren nach Anspruch 13, in dem der Nitroarylessigsäureester ein Alkyl-2-(3-Halo-4-nitrobenzol)propionat ist.

15. Verfahren zur Herstellung von Alkyl 2-(4-aminobenzol)propionat, das die Verfahrensstufen eines Verfahrens nach Anspruch 13 aufweist, unter Bildung von Alkyl-2-(4-nitrobenzol)propionat oder Alkyl-2-(3-chlor-4-nitrobenzol)propionat und in dem das erhaltene Produkt unter Bildung von Alkyl-2-(4-aminobenzol)propionat hydriert wird.

16. Verfahren zur Herstellung von Alkyl-2-(4-phthalimidophenyl)propionat, das die Verfahrensstufen eines Verfahrens nach Anspruch 15 aufweist und in dem das erhaltene Alkyl-2-(4-aminobenzol)propionat mit Phthalsäureanhydrid zur Reaktion gebracht wird.

17. Verfahren zur Herstellung von 2-[4-(1-,3-Dihydro-1-oxo-2H-isoindol-2-yl)phenyl]propionsäure, das die Verfahrensstufen eines Verfahrens nach Anspruch 16 aufweist und in dem das erhaltene Alkyl-2-(4-phthalimidophenyl)propionat reduziert und hydrolisiert wird.

18. Verfahren zur Herstellung von 2-(4-Nitrobenzol)propionsäure, das die Verfahrensstufen eines Verfahrens nach Anspruch 13 zur Herstellung von Alkyl-2-(4-nitrobenzol)propionat aufweist und in dem das erhaltene Produkt hydrolisiert wird.

19. Verfahren nach Anspruch 18, in dem das Säureprodukt entweder 2-(4-Nitrobenzol)propionsäure ist oder das Alkyl-2-(4-nitrobenzol)propionat ist. Alkyl-2-(3-chlor-4-nitrobenzol)propionat und das Säureprodukt ist 2-(3-Chlor-4-nitrobenzol)propionsäure.

20. Verfahren zur Herstellung von 2-(Aminobenzol)propionsäure, das die Verfahrensstufen eines Verfahrens nach Anspruch 19 zur Herstellung von 2-(4-Nitrobenzol)propionsäure oder 2-(3-Chlor-4-nitrobenzol)propionsäure aufweist und in dem die erhaltene Säure reduziert wird.

21. Verfahren zur Herstellung von 2-(4-Phthalimidophenyl)propionsäure, das die Verfahrensstufen eines Verfahrens nach Anspruch 20 aufweist in dem die erhaltene 2-(4-Aminobenzol)propionsäure mit Phthalsäureanhydrid umgesetzt wird.

22. Verfahren zur Herstellung von 2-[4-(1,3-Dihydro-1-oxo-2H-isoindol-2-yl)phenyl]propionsäure, das die Verfahrensstufen eines Verfahrens nach Anspruch 21 aufweist und in dem die erhaltene 2-(4-Phthalimidophenyl)propionsäure reduziert wird.

23. Verfahren zur Herstellung von Alkyl-2-(4-amino-3-chlor oder 3-Fluorbenzol)propionat, das die

Verfahrensstufen eines Verfahrens nach Anspruch 14 zur Herstellung eines Alkyl-2-(3-chlor oder 3-Fluor-4-nitrobenzol)propionats aufweist, und in dem das erhaltene Produkt selektiv hydriert wird.

24. Verfahren zur Herstellung von Alkyl-2-[3-chlor-4-(3-pyrrolinyl)phenyl]propionat, das die Verfahrensstufen eines Verfahrens nach Anspruch 23 zur Herstellung eines Alkyl-2-(4-amino-3-chlorbenzol)propionats aufweist und in dem das erhaltene Produkt mit einem 1,4 Dihalo-2-buten umgesetzt wird.

25. Verfahren zur Herstellung von 2-[3-Chlor-4-(3-pyrrolinyl)phenyl]propionsäure, das die Verfahrensstufen eines Verfahrens nach Anspruch 24 aufweist und in dem das erhaltene Alkyl-2-[3-chlor-4-(3-pyrrolinyl)phenyl]propionat hydrolisiert wird.

26. Verfahren zur Herstellung von Alkyl-2-(2-fluor-4-biphenylyl)propionat, das die Verfahrensstufen eines Verfahrens nach Anspruch 23 zur Herstellung von Alkyl-2-(4-amino-3-fluorbenzol)propionat aufweist und in dem das erhaltene Produkt einer Gomberg-Bachmann-Reaktion unterworfen wird um die Aminogruppe durch ein Phenylgruppe zu ersetzen.

27. Verfahren zur Herstellung von 2-(2-Fluor-4-biphenylyl)propionsäure, das die Verfahrensstufen eines Verfahrens nach Anspruch 26 aufweist und in dem das erhaltene Alkyl-2-(2-fluor-4-biphenylyl)-propionat hydrolisiert wird.

28. Verfahren zur Herstellung einer pharmazeutischen oder veterinärmedizinischen Zubereitung, bei der man 2-(2-Fluor-4-biphenylyl)propionsäure oder 2-[3-Chlor-4-(3-pyrrolinyl)phenyl]propionsäure oder 2-[4-(1,3-Dihydro-1-oxo-2H-isoindol-2yl)phenyl]propionsäure nach einem Verfahren nach Anspruch 27, Anspruch 25 oder entweder gemäß den Ansprüchen 17 und 22 herstellt, und die erhaltene Säure für eine pharmazeutische oder veterinärmedizinische Verwendung formuliert.

**Revendications**

1. Procédé de préparation d'un ester d'acide nitroarylacétique, qui consiste à faire réagir un composé aromatique nitré avec un ester d'acide acétique alpha,alpha-disubstitué dans un solvant aprotique quasi anhydre et en présence d'une base.

2. Procédé suivant la revendication 1, dans lequel le composé aromatique nitré est dépourvu d'halogène sur le noyau aromatique portant le groupe nitro.

3. Procédé suivant la revendication 1, dans lequel le composé aromatique nitré est un composé aromatique nitré halogéné portant un substituant halogéné sur le noyau aromatique porteur du groupe nitro.

4. Procédé suivant la revendication 2 ou la revendication 3, dans lequel le composé aromatique nitré est un mononitrobenzène présentant une position non substituée en para par rapport au groupe nitro.

5. Procédé suivant la revendication 4, dans lequel le mononitrobenzène est le nitrobenzène ou un o-halogénonitrobenzène.

6. Procédé suivant la revendication 1, dans lequel le composé aromatique nitré est un chloronitrobenzène, de préférence un o-chloronitrobenzène.

7. Procédé suivant la revendication 1, dans lequel le composé aromatique nitré est un composé aromatique nitré fluoré, de préférence un o-fluoronitrobenzène.

8. Procédé suivant la revendication 1, dans lequel le composé aromatique nitré est un composé aromatique nitré halogéné dont le substituant halogéno a un nombre atomique au moins égal à 17, l'ester d'acide nitroarylacétique résultant étant un ester d'acide halogénonitroarylacétique.

9. Procédé suivant l'une quelconque des revendications 1 à 8, dans lequel l'ester d'acide acétique alpha,alpha-disubstitué est un ester d'acide alpha-halogénomonocarboxylique contenant au moins trois atomes de carbone dans la portion acide.

10. Procédé suivant l'une quelconque des revendications 1 à 8, dans lequel l'ester d'acide acétique alpha,alpha-disubstitué est un alpha-chloro- ou alpha-bromopropionate d'alkyle.

11. Procédé suivant l'une quelconque des revendications 1 à 10, dans lequel le solvant est un solvant aprotique dipolaire.

12. Procédé suivant l'une quelconque des revendications 1 à 11, dans lequel la base est un composé de métal alcalin, de préférence l'hydrure de sodium ou l'hydrure de potassium.

13. Procédé suivant l'une quelconque des revendications 1 à 12, dans lequel l'ester d'acide nitroarylacétique est un ester d'acide 2-(4-nitrobenzène)acétique ou un 2-(4-nitrobenzène)propionate d'alkyle.

14. Procédé suivant la revendication 13, dans lequel l'ester d'acide nitroarylacétique est un 2-(3-halogéno-4-nitrobenzène)propionate d'alkyle.

15. Procédé de préparation d'un 2-(4-aminobenzène)propionate d'alkyle, procédé qui consiste à effectuer les étapes d'un procédé suivant la revendication 13 pour produire un 2-(4-nitrobenzène)-propionate d'alkyle ou un 2-(3-chloro-4-nitrobenzène)propionate d'alkyle, et à soumettre le produit résultant à une hydrogénation pour former un 2-(4-aminobenzène)propionate d'alkyle.

16. Procédé de préparation d'un 2-(4-phtalimidophényl)propionate d'alkyle, qui consiste à effectuer les étapes d'un procédé suivant la revendication 15 et à faire réagir le 2-(4-aminobenzène)propionate d'alkyle résultant avec l'anhydride phtalique.

17. Procédé de préparation de l'acide 2-[4-(1,3-dihydro-1-oxo-2H-isoindol-2-yl)phényl]propionique, qui

consiste à effectuer les étapes d'un procédé suivant la revendication 16 et à soumettre le 2-(4-phtalimidophényl)propionate d'alkyle résultant à une réduction et à une hydrolyse.

18. Procédé de préparation d'un acide 2-(4-nitrobenzène)propionique, qui consiste à effectuer les étapes d'un procédé suivant la revendication 13 pour produire un 2-(4-nitrobenzène)propionate d'alkyle et à hydrolyser le produit résultant.

19. Procédé suivant la revendication 18, dans lequel l'acide obtenu comme produit est l'acide 2-(4-nitrobenzène)propionique ou bien le 2-(4-nitrobenzène)propionate d'alkyle est un 2-(3-chloro-4-nitrobenzène)propionate d'alkyle et l'acide obtenu comme produit est l'acide 2-(3-chloro-4-nitrobenzène)propionique.

20. Procédé de préparation d'acide 2-(aminobenzène)propionique, qui consiste à effectuer les étapes d'un procédé suivant la revendication 19 pour produire l'acide 2-(4-nitrobenzène)propionique ou l'acide, 2-(3-chloro-4-nitrobenzène)propionique et à réduire l'acide résultant.

21. Procédé de préparation de l'acide 2-(4-phtalimidophényl)propionique, qui consiste à effectuer les étapes d'un procédé suivant la revendication 20 et à faire réagir l'acide 2-(4-aminobenzène)propionique résultant avec l'anhydride phtalique.

22. Procédé de préparation de l'acide 2-[4-(1,3-dihydro-1-oxo-2H-isoindol-2-yl)phényl]propionique, qui consiste à effectuer les étapes d'un procédé suivant la revendication 21, et à réduire l'acide 2-(4-phtalimidophényl)propionique résultant.

23. Procédé de préparation d'un 2-(4-amino-3-chloro ou 3-fluorobenzène)propionate d'alkyle, qui consiste à effectuer les étapes d'un procédé suivant la revendication 14 pour produire un 2-(3-chloro ou 3-fluoro-4-nitrobenzène)propionate d'alkyle et à hydrogéner sélectivement le produit résultant.

24. Procédé de préparation d'un 2-[3-chloro-4-(3-pyrrolinyl)phényl]propionate d'alkyle, qui consiste à effectuer les étapes d'un procédé suivant la revendication 23 pour produire un 2-(4-amino-3-chlorobenzène)propionate d'alkyle et à faire réagir le produit résultant avec un 1,4-dihalogéno-2-butène.

25. Procédé de préparation de l'acide 2-[3-chloro-4-(3-pyrrolinyl)phényl]propionique, qui consiste à effectuer les étapes d'un procédé suivant la revendication 24 et à hydrolyser le 2-[3-chloro-4-(3-pyrrolinyl)-phényl]propionate d'alkyle résultant.

26. Procédé de préparation d'un 2-(2-fluoro-4-biphénylyl)propionate d'alkyle, qui consiste à effectuer les étapes du procédé suivant la revendication 23, pour produire un 2-(4-amino-3-fluorobenzène)-propionate d'alkyle et à soumettre le produit résultant à une réaction de Gomberg-Bachmann pour remplacer le groupe amino par un groupe phényle.

27. Procédé de préparation de l'acide 2-(2-fluoro-4-biphénylyl)propionique, qui consiste à effectuer les étapes d'un procédé suivant la revendication 26 et à hydrolyser le 2-(2-fluoro-4-biphénylyl)propionate d'alkyle résultant.

28. Procédé de préparation d'une formulation pharmaceutique ou vétérinaire, qui consiste à préparer l'acide 2-(2-fluoro-4-biphényl)propionique ou l'acide 2-[3-chloro-4-(3-pyrrolinyl)phényl]propionique ou l'acide 2-[4-(1,3-dihydro-1-oxo-2H-isoindol-2-yl)phényl]propionique par un procédé suivant la revendication 27, la revendication 25 ou respectivement l'une des revendications 17 et 22, et à formuler l'acide résultant en vue d'un usage pharmaceutique ou respectivement vétérinaire.